# EUROPÄISCHE PATENTSCHRIFT

(11) **EP 0 744 214 B1**
(45) Veröffentlichungstag und Bekanntmachung des Hinweises auf die Patenterteilung: **14.07.1999**
(21) Anmeldenummer: 96108216.1
(22) Anmeldetag: 23.05.1996
(51) Int. Cl.: B01J 23/22, B01J 27/198, C07C 51/31, C07C 63/16

(54) **Trägerkatalysator für Gasphasenoxidationsreaktionen**
Supported catalyst for oxidation reactions in the gas phase
Catalyseur sur support pour les réactions d'oxydation en phase gazeuse

(30) Priorität: 24.05.1995 DE 19519172
(43) Veröffentlichungstag der Anmeldung: 27.11.1996
(73) Patentinhaber: Consortium für Elektrochemische Industrie GmbH, 81379 München (DE)
(72) Erfinder: Eberle, Hans-Jürgen, Dr., 81477 München (DE); Wagner, Werner, Dr., 81541 München (DE); Grundei, Franz, 85560 Ebersberg (DE); Liebisch, Erich, 80798 München (DE)
(74) Vertreter: Schuderer, Michael, Dr.

(56) Entgegenhaltungen:
- EP-A- 0 017 865
- FR-A- 2 303 594
- FR-A- 2 442 843
- GB-A- 2 067 088

## Beschreibung

Die Erfindung betrifft Trägerkatalysatoren für Gasphasenoxidationsreaktionen, Verfahren zu deren Herstellung sowie deren Verwendung bei Gasphasenoxidationsreaktionen.

Bei der Gasphasenoxidation von Kohlenwasserstoffen wird üblicherweise ein Gemisch aus Kohlenwasserstoff mit Luft bzw. mit sauerstoffhaltigen Gasen über einen Katalysator geleitet, der sich beispielsweise in einem Röhrenreaktor befindet, welcher aus einer Vielzahl von mit Katalysator gefüllten Röhren besteht. Zur Beheizung auf die erforderliche Reaktionstemperatur wie auch zur Kühlung dieser stark exotherm verlaufenden Reaktionen sind die Röhren von einer Salzschmelze umgeben. Bei diesen (exotherm verlaufenden) Oxidationsverfahren werden allerdings mit zunehmender Beladung der Luft mit den zu oxidierenden Kohlenwasserstoffen vermehrt Nebenprodukte durch Totaloxidation gebildet. Grund hierfür sind im Katalysatorbett auftretende Temperaturspitzen, die sogenannten Hot Spots, die um so hoher sind, je größer die Beladung der Luft mit den zu oxidierenden Kohlenwasserstoffen ist. Diese Hot Spots können so groß werden, daß es dadurch zu dem sogenannten runaway-Verhalten des Reaktors kommt, was zu einer Schädigung bzw. Desaktivierung des Katalysators führt.
Es gab daher bereits in der Vergangenheit eine Vielzahl von Anstrengungen, die Selektivität der Gasphasenoxidation durch die Verwendung spezieller Katalysatoren zu steigern.

Trägerkatalysatoren für die Gasphasenoxidation von Kohlenwasserstoffen zu Carbonsäureanhydriden, deren katalytisch aktive Oberflächenbeschichtung im wesentlichen aus Titandioxid (TiO₂), bevorzugt in der Anatas-Modifikation, und Divanadiumpentoxid (V₂O₅) besteht, sind seit längerem bekannt und haben gerade bei so exothermen Reaktionen wie der Oxidation von o-Xylol und/oder Naphthalin zu Phthalsäureanhydrid (PSA) gegenüber entsprechend zusammengesetzten trägerfreien (homogenen) Kontakten eine Reihe von Vorteilen, wie höhere Selektivität und besseres Betriebsverhalten.

So ist in der US-A 2,157,965 die Oxidation von Naphthalin zu PSA mit Katalysatoren beschrieben, die durch Aufsprühen einer Mischfällung aus einer wäßrigen Lösung bzw. Suspension, die Ammoniummetavanadat und Titanylsulfat enthielt, auf Träger wie Bimsstein und anschließender Calcinierung hergestellt wurden. Die Nachteile dieser Katalysatoren sind der anfangs hohe Anteil an Totaloxidation und deren geringe Lebensdauer.

Durch Verwendung inerter, nicht poröser Träger wie z.B. Magnesiumsilikat oder Porzellan und direktes Aufsprühen von TiO₂-haltigen Suspensionen, die als Vanadinkomponente Vanadyloxalat enthielten (DE-PS 14 42 590 = US-A 3,509,179), und durch Verwendung zweier verschiedener TiO₂-Komponenten, von denen eine Anatas-Komponente eine BET-Oberfläche von 7-11 m²/g und eine Titandioxidhydrat-Komponente eine BET-Oberfläche von über 100 m²/g aufweist (DE-C3 21 06 796 = US-A 3,799,886), konnten die Selektivität der Oxidationsreaktion und die Lebensdauer der Katalysatoren verbessert werden.

Auch durch die Verwendung von Zweischichtfüllungen im Röhrenreaktor konnte die max. Beladung nur leicht gesteigert werden. Dazu wurde die katalytische Aktivität der Oberschicht durch Zusatz von Alkalimetallen gedämpft, die Unterschicht dagegen durch Phosphor-Dotierung aktiviert. In der DE-AS 25 46 268 (= US-A 4,077,984) sind bei dieser Verfahrensweise Beladungssteigerungen bei der Oxidation von o-Xylol zu Phthalsäureanhydrid von 42 auf lediglich 60 g/Nm³ Luft beschrieben. Ähnliches wurde bei der Naphthalin-Oxidation beobachtet.

In der DE-PS 29 48 163 (US-A 4,284,571) ist ein Katalysator beschrieben, dessen katalytische aktive Masse, bestehend aus V₂O₅, TiO₂-Anatas und verschiedenen Promotoren wie Oxiden von Phosphor, Niob, Cäsium und/oder Thallium, auf einem porösen Träger aufgebracht ist, der mindestens zu 80% aus Siliciumcarbid besteht. Der Vorteil dieses Trägerkatalysators besteht im Absenken der Hot-Spot-Temperatur durch Verwendung einer aus Ilmenit gewonnen TiO₂-Komponente (BET-Oberfläche: 10-60 m²/g).

Aus der DE-PS 30 45 624 (= US-A 4,356,112) sind Katalysatoren mit verbesserter Wärmestabilität bekannt, welche die Hot-Spot-Temperatur bei der Oxidation von o-Xylol oder Naphthalin absenken. Die Katalysatoren sind Trägerkatalysatoren, welche eine Aktivschicht auf Basis TiO₂/V₂O₅ mit Nb₂O₅, Cs₂O, K₂O, P₂O₅ und Sb₂O₃ auf einem porösen Träger auf Siliciumcarbid-Basis aufweisen, wobei durch den Sb₂O₃-Gehalt die Wärmestabilität erhöht wird. Ein ähnlicher Katalysator zur Herstellung von Pyromellithsäuredianhydrid (PMDA) ist in der EP-B 163 231 beschrieben. Die zuletzt genannten Katalysatoren werden vor deren Einsatz calciniert und besitzen dementsprechend eine relativ geringe Abriebfestigkeit, was zum Verlust an katalytisch aktiver Oberflächenbeschichtung beim Einfüllen in industrielle Reaktoren führen kann. Zur Verbesserung der Haftung der Aktivschicht auf dem porösen SiC-Träger wird empfohlen, der Aktivschicht metallische oder keramische Whiskers mit definierten geometrischen Abmessungen zuzumischen. Alle diese Kontakte auf Basis poröser SiC-Träger haben den Nachteil des hohen Preises und der schwierigen Wiederverwertung des Trägers.

Die der Erfindung zugrundeliegende Aufgabe bestand daher darin, für industrielle Reaktoren geeignete, verbesserte Trägerkatalysatoren für die Luftoxidation, speziell von aromatischen Kohlenwasserstoffen, insbesondere für die Oxidation von Naphthalin oder einem Gemisch aus Naphthalin und o-Xylol zu PSA oder für die Oxidation von 1,2,4,5-Tetramethylbenzol (Durol) zu PMDA zu finden, die das gewünschte Reaktionsprodukt in hohen Ausbeuten liefern. Ein besonderer Schwerpunkt der Aufgabe lag darin, Katalysatoren zu entwickeln, die hohe Kohlenwasserstoffbeladungen der Reaktionsluft, wenn möglich auch schon in der Anfangsphase, ohne Schädigungen des Katalysators umsetzen können, so daß auch bei hohen Beladungen eine Hot-Spot-Temperatur (heißeste Temperatur im Katalysatorbett) von 500°C nicht überschritten wird.

Überraschenderweise konnte diese Aufgabe mit einem Trägerkatalysator gelöst werden aus nicht porösem, inertem Trägerkörper und einer Oberflächenbeschichtung, bei der ein Teil der TiO₂-Aktivkomponente durch SiC-Pulver substituiert wurde.

Gegenstand der Erfindung ist ein Trägerkatalysator für Gasphasenreaktionen mit einem inerten Trägerkörper und einer Oberflächenbeschichtung enthaltend
a) mindestens 5 Gew.% Siliciumcarbid,
b) 5 bis 90 Gew.%, gerechnet als Oxid, einer oder mehrerer Titandioxid- oder Zirkonoxid-Komponenten oder deren Gemische,
c) 1 bis 50 Gew.%, gerechnet als V₂O₅, einer oder mehrerer Vanadiumoxidkomponenten,
d) 0 bis 10 Gew.%, gerechnet als Oxid, einer oder mehrerer Verbindungen von Elementen der 1. und 5. Hauptgruppe des Periodensystems,
   wobei die Angaben in Gewichtsprozent jeweils auf das Gesamtgewicht der Aktivkomponenten bezogen sind und sich auf 100 Gew.% aufaddieren.

Im Prinzip können die Trägerkörper beliebige Gestalt und Oberflächenstruktur besitzen. Bevorzugt werden jedoch regelmäßig geformte, mechanisch stabile Körper wie Kugeln, Ringe, Halbringe, Sättel, oder wabenförmige oder mit Kanälen versehene Träger. Die Größe der Trägerkörper wird vorwiegend von der Dimension, vor allem vom inneren Durchmesser der Reaktionsrohre bestimmt, wenn der Katalysator in Röhren- bzw. Rohrbündelreaktoren zur Anwendung kommt. Der Trägerdurchmesser sollte dann zwischen 1/2 und 1/10 des Innendurchmessers betragen. Als Materialien eignen sich beispielsweise Steatit, Duranit, Steingut, Porzellan, Siliciumdioxid, Silicate, Aluminiumoxid, Aluminate oder Mischungen aus diesen Stoffen. Vorzugsweise werden Kugeln oder Ringe aus Trägermaterialien wie Duranit oder Steatit eingesetzt.

Der Anteil der Oberflächenbeschichtung mit aktiver Masse beträgt 1-30 Gew.%, vorzugsweise 2-15 Gew.%, bezogen auf die Gesamtmasse des Trägerkatalysators. Die Schichtdicke der Oberflächenbeschichtung beträgt vorzugsweise 10 bis 120µm. Als Komponente a) der Oberflächenbeschichtung eignet sich handelsübliches Siliciumcarbid-Pulver mit einer Korngröße von vorzugsweise bis 100 µm, wobei möglichst feines SiC-Pulver bevorzugt wird. Sehr gute Ergebnisse erhält man beispielsweise mit SiC mit einer Korngröße von 10 bis 50 nm. Bevorzugt wird ein Anteil von 10 bis 75 Gew.%, insbesondere 30 bis 75 Gew.% Siliciumcarbid, bezogen auf das Gesamtgewicht der Aktivkomponenten.

Als Komponente b) wird vorzugsweise pulverförmiges TiO₂ in der Anatas-Modifikation mit einer BET-Oberfläche von 5 bis 200 m²/g eingesetzt. Bevorzugt wird auch ein Titandioxidhydrat (hydroxylgruppenreicher, mikrokristalliner Anatas) mit einer BET-Oberfläche von mehr als 100 m²/g oder ein Gemisch von Anatas mit einer BET-Oberfläche von 5-11 m²/g und Titandioxidhydrat mit einem Mischungsverhältnis von vorzugsweise 1:3 bis 3:1 eingesetzt. Bevorzugt wird ein Anteil von 10 bis 75 Gew.%, insbesondere 20 bis 65 Gew.% Komponente b) eingesetzt, gerechnet als Oxid und bezogen auf das Gesamtgewicht der Aktivkomponenten.

Als Komponente c) können Vanadiumoxide oder Vanadiumverbindungen, die sich beim Erhitzen an der Luft in Vanadiumoxid umwandeln, einzeln oder in Form deren Gemische eingesetzt werden. Vorzugsweise werden V₂O₅ oder NH₄VO₃ eingesetzt. Bevorzugt wird ein Anteil von 5 bis 30 Gew.% Vanadiumoxid-Komponente eingesetzt, gerechnet als V₂O₅ und bezogen auf das Gesamtgewicht der Aktivkomponenten.

Geeignete Komponenten d) sind beispielsweise Alkalimetallverbindungen wie K₂O, Cs₂O, Cs₂CO₃ in einer Menge von vorzugsweise 0,01 bis 1,0 Gew.%, jeweils bezogen auf das Gesamtgewicht der Aktivkomponenten. Geeignet sind auch Verbindungen von Phosphor, Antimon, Wismuth, vorzugsweise deren Oxide, in einer Menge von vorzugsweise 0,1 bis 10 Gew.%, jeweils bezogen auf das Gesamtgewicht der Aktivkomponenten. Besonders bevorzugte Beispiele für die zuletztgenannte Gruppe sind P₂O₅, (NH₄)₂HPO₄, Sb₂O₃.

Zur Herstellung der Trägerkatalysatoren werden die Trägerkörper vorzugsweise mit einer wäßrigen Aufschlämmung einer Mischung der Aktivkomponenten oder aber der einzelnen Komponenten, beispielsweise in einem Drehrohrofen bei 200-300°C, beschichtet und getrocknet. Trägerkatalysatoren mit sehr gut haftenden Überzügen, was insbesondere für den Transport und das Einfüllen des Katalysators in den Reaktor von Bedeutung ist, erhält man beispielsweise, indem eine wäßrige Suspension, welche die Mischung oder die einzelnen Komponenten sowie gegebenenfalls einen organischen Binder enthält, auf die Trägerkörper gleichmäßig aufgebracht wird. Als organische Binder bevorzugt sind Copolymere, vorteilhaft in Form einer wäßrigen Dispersion, von Vinylacetat/Vinyllaurat, Vinylacetat/Acrylat, Styrol/Acrylat, Vinylacetat/Maleat sowie Vinylacetat/Ethylen. Bindermengen von 10-20 Gew.%, bezogen auf den Feststoffgehalt der Suspension, sind völlig ausreichend. Diese Copolymere brennen nach dem Einfüllen des Katalysators in den Reaktor innerhalb kurzer Zeit im Luftstrom quantitativ heraus.

Die Trägerkatalysatoren eignen sich beispielsweise zur Verwendung als Oxidationskatalysatoren bei der Oxidation von Aromaten oder Alkylaromaten sowie deren Gemische zur Herstellung der entsprechenden Säureanhydride, vorzugsweise zur Herstellung von Phthalsäureanhydrid (PSA) durch katalytische Gasphasenoxidation von o-Xylol oder Naphthalin oder Gemischen aus o-Xylol und Naphthalin. Eine weitere bevorzugte Verwendung ist die als Oxidationskatalysator bei der Herstellung von Pyromellithsäuredianhydrid (PMDA) durch katalytische Gasphasenoxidation von 1,2,4,5-tetraalkylierten Benzolen (beispielsweise Durol = 1,2,4,5-Tetramethylbenzol).

Bei der Herstellung von PSA und PMDA werden die jeweiligen Edukte zusammen mit einem sauerstoffhaltigen Gas, in Gegenwart des erfindungsgemäßen Katalysators, vorzugsweise in Festbettreaktoren umgesetzt. Übliche Festbettreaktoren sind beispielsweise Reaktionsrohre, die zu Rohrbündelreaktoren zusammengefaßt und von einem Wärmetauschermedium umgeben sind. Die Reaktionsrohre sind vertikal angeordnet und werden vom Reaktionsgemisch von oben her durchströmt. Sie bestehen aus einem gegenüber Wärmetauschermedium, Katalysator, Edukten und Produkten inertem Material, im allgemeinen Stahl, und besitzen eine Länge von 2000 bis 6000 mm, einen Innendurchmesser von 10 bis 30 mm und eine Wandstärke von 1 bis 4 mm. Als Wärmetauschermedien haben sich in der Praxis eutektische Salzgemische bewährt, beispielsweise eine chloridfreie Schmelze aus Kaliumnitrat und Natriumnitrit.

Der Katalysator wird von oben in die Reaktionsrohre eingefüllt und durch in der Nähe der unteren Rohrenden angebrachte Halterungen fixiert. Die Füllhöhe kann zwischen 900 und 3300 mm betragen. Die Reaktionsrohre können gegebenenfalls schichtweise mit Trägerkörpern unterschiedlicher Gestalt und Dimension sowie unterschiedlicher Konzentration und Zusammensetzung der Aktivkomponenten befüllt werden.

Das Reaktionsgas, bestehend aus Edukt-Kohlenwasserstoffen und einem sauerstoffhaltigen Gas, vorzugsweise Luft, wird mit einer Raumgeschwindigkeit von 800 bis 8000 h⁻¹, vorzugsweise 1000 bis 6000 h⁻¹, über den Katalysator geleitet. Dabei beträgt das Mischungsverhältnis 10 bis 150 g Kohlenwasserstoff pro Normkubikmeter sauerstoffhaltigem Gas.

Nach der Umsetzung wird das gebildete Produkt in an sich bekannter Weise durch Desublimation oder durch entsprechende Gaswäsche mit einem geeigneten Lösungsmittel aus dem Reaktionsgas gewonnen.

Die erfindungsgemäßen Trägerkatalysatoren unterscheiden sich von den bisher bekannten Oxidationskatalysatoren auf TiO₂/V₂O₅-Basis dadurch, daß ein Teil der TiO₂-Komponente durch SiC ausgetauscht wird.

Es zeigte sich, daß sich die Zumischung von SiC-Pulver zur katalytisch aktiven Oberflächenbeschichtung von Katalysatoren besonders vorteilhaft auf das Betriebsverhalten und die Selektivität von Katalysatoren auswirkt. Insbesondere trifft dies für die Oxidation von Naphthalin oder von einem Gemisch aus Naphthalin und o-Xylol zu PSA und für die Oxidation von Durol zu PMDA zu. Mit SiC-haltigen Katalysatoren werden sowohl bei der Naphthalin- und/oder o-Xylol-Oxidation als auch bei der Durol-Oxidation höhere carbonsäureanhydrid-Ausbeuten bei höheren Kohlenwasserstoffbeladungen erzielt.

Völlig unerwartet wurden durch Austausch eines Teils der TiO₂-Aktivkomponenten mit dem bisher nur als inerten Füllstoff bekannten SiC Katalysatoren erhalten, die den bisher verwendeten SiC-freien bezüglich Selektivität und maximaler Beladung überlegen sind. Mit den erfindungsgemäßen Katalysatoren sind höhere Beladungen möglich. Die Ausbeuten werden deutlich verbessert. Die erfindungsgemäßen Trägerkatalysatoren reagieren unempfindlich gegen kurzzeitige Belastung bei Temperaturen über 600°C.

Die nachfolgenden Beispiele dienen zur weiteren Erläuterung der Erfindung.

### Katalysatorpräparation

Die Aktivkomponenten wurden in den in Tabelle 1 angegebenen Mengen in 400 ml entionisiertem Wasser suspendiert und 18 Stunden gerührt, um eine homogene Verteilung zu erzielen. Vor dem Auftragen der Mischung auf die in Tabelle 1 angegebenen Steatit-Trägerkörper wurde der Suspension der organische Binder, ein Copolymeres aus Vinylacetat und Vinyllaurat, in Form einer 50%igen wäßrigen Dispersion zugegeben.

Anschließend wurde die Suspension unter Verdampfen des Wassers auf den Träger aufgebracht.

**Tabelle 1**

| Zusammensetzung der Katalysatoren: | | | | | |
|---|---|---|---|---|---|
| **Katalysator** | **A** | **B** | C | D | **E** |
| Träger | 7x4x4 mm Ringe 1225 g | 7x4x4 mm Ringe 1225 g | 7x4x4 mm Ringe 1225 g | 8 mm Kugeln 1000 g | 8 mm Kugeln 1000 g |
| V₂O₅ | 15.33 g | 15.12 g | 15.12 g | 19.6 g | 19.6 g |
| TiO₂-Hydrat BET-Oberfläche: > 150 m²/g | 22.29 g | 23.67 g | 23.67 g | 40.27 g | 40.27 g |
| SiC Korn-⌀ | - | **94.69 g 4 µm ⌀** | **94.69 g 30 nm ⌀** | - | **16.00 g 4 µm ⌀** |
| Anatas BET-Oberfläche: < 10 m²/g | **96.00 g** | - | - | **16.00 g** | - |
| Steatit-Mehl Korn-⌀ | - | - | - | - | - |
| Cs₂CO₃ | 322,7 mg | 393.6 mg | 393.6 mg | - | - |
| (NH₄)₂HPO₄ | - | - | - | 6.35 g | 6.35 g |
| Dispersion | 42 g | 42 g | 42 g | 30 g | 30 g |

Zur Testung der Eignung der Trägerkatalysatoren als Oxidationskatalysatoren wurden diese bei der Naphthalin-Oxidation zu Phthalsäureanhydrid (Beispiele 1 und 2) und bei der Durol-Oxidation zu PMDA (Beispiel 3) getestet. Als Vergleich wurden herkömmliche Katalysatoren auf Basis TiO₂/V₂O₅ (Vergleichsbeispiel 1, 2) eingesetzt.

Die Oxidationsversuche wurden in einem technischen Maßstäben nachempfundenen Reaktionsrohr durchgeführt. Die Länge des Reaktionsrohres betrug 3,3 m (Füllhöhe 2,8 m), dessen Durchmesser 25 mm. Der Reaktor wurde mit einem umgewälzten Salzbad (eutektische, chloridfreie Schmelze aus Kaliumnitrat und Natriumnitrit) temperiert. Die eingespeiste Luftmenge lag bei 4 Nm³/h. Die Reinheit der Edukte lag immer über 99%.

**Tabelle 2**

| Ergebnisse der Oxidationsversuche: Vergleich Anatas - SiC | | | | | |
|---|---|---|---|---|---|
| **Beispiel** | Vgl.bsp.1 | Bsp. 1 | Bsp.2 | Vgl.bsp.2 | Bsp.3 |
| **Katalysator** | **A** (Anatas) | **B** (SiC) | C (SiC) | D (Anatas) | E (SiC) |
| Edukt | N ⁴) | N | N | D ⁵) | D |
| MV (max) ¹) [g/Nm³] | 52 | 80 | 90 | 26 | 40 |
| SBT [°C] ²) | 360 | 365 | 364 | 376 | 385 |
| HST [°C] ³) | 470 | 450 | 446 | 482 | 461 |
| Reinausbeute [Gew.-%] | 98 PSA | 101 PSA | 100 PSA | 80 PMDA | 80 PMDA |

| | | | | | |
|---|---|---|---|---|---|
| ¹) MV (max) ist die maximal einstellbare Kohlenwasserstoffbeladung der Luft in g Kohlenwasserstoff pro Nm³ Luft. | | | | | |
| ²) Salzbad-Temperatur | | | | | |
| ³) Hot-Spot-Temperatur | | | | | |
| ⁴) Naphthalin | | | | | |
| ⁵) Durol | | | | | |

Die Beispiele 1 und 2 zeigen die Vorteile von SiC bei der Naphthalin-Oxidation zu Phthalsäureanhydrid. Gegenüber dem Anatas-haltigen Katalysator A (Vgl.bsp. 1) wurde mit Katalysator B (Bsp. 1) die PSA-Ausbeute um 3 Gew.% verbessert.

Gleichzeitig waren mit Katalysator B (Beispiel 1) wesentlich höhere Naphthalin-Beladungen bei niedrigeren Hot-Spot-Temperaturen möglich.

Mit sehr feinkörnigem SiC (30 nm Korndurchmesser, Beispiel 2) waren sogar Beladungen bis MV 90 bei noch niedrigerer HST möglich. Beispiel 2 beweist die große Flexibilität bei der Wahl der SiC-Korngröße und die Verbesserung des Effektes bei abnehmender Korngröße.

Auch bei der Durol-Oxidation (Vgl.bsp.2, Bsp. 3) waren deutlich höhere Beladungen bei niedrigeren Hot-Spot-Temperaturen möglich.

**Tabelle 3**

| Zusammensetzung der Katalysatoren: Vergleich Anatas - SiC bei Zweischicht-Füllungen | | | |
|---|---|---|---|
| **Katalysator** | F | G | H |
| Träger | 7x7x4 mm Ringe 1000 g | 7x7x4 mm Ringe 1000 g | 7x7x4 mm Ringe 1000 g |
| Füllung Füllhöhe | Oberfüllung 150 cm | Oberfüllung 150 cm | Unterfüllung 130 cm |
| V₂O₅ | 9,95 g | 10,23 g | 10,05 g |
| Ti-Hydrat | 17,60 g | 16,02 g | 19,63 g |
| SiC (Korn-⌀) | - | **38,45 g (4 µm ⌀)** | - |
| Anatas BET<10m²/g | **59,19 g** | 25,63 g | **56,08 g** |
| Cs₂CO₃ | 220 mg | 222 mg | - |
| (NH₄)₂HPO₄ | - | - | 1,372 g |
| Dispersion | 35 g | 35 g | 35 g |

Auch bei den in der Technik mittlerweile üblichen Zweischicht-Füllungen aus gedämpfter Oberschicht und aktivierter Unterschicht beweist SiC seine Vorteile. Dabei kann es ausreichen, nur die Oberfüllung, in der sich die Hot-Spot-Zone befindet, mit SiC Zu versehen (Katalysator G). Dieser Katalysator G (Oberschicht) bei dem 60% der oberflächenarmen Anatas-Komponente gegen SiC ausgetauscht wurden, konnte nach ca. 4 Wochen Betriebsdauer mit 102 g/Nm³ beladen werden, ohne daß Hot-Spot-Temperaturen über 470°C auftraten (Beispiel 4). Für Beispiel 4 und Vergleichsbeispiel 3 wurde jeweils die gleiche Unterschicht H verwendet.

**Tabelle 4**

| Ergebnisse der Oxidationsversuche: Vergleich Anatas - SiC/Anatas bei Zweischicht-Füllungen | | |
|---|---|---|
| **Beispiel** | Vgl.bsp. 3 | Beispiel 4 |
| **Katalysator** | F / **H** (Anatas) | G / **H** (SiC/Anatas) |
| Edukt | N | N |
| MV (max) [g/Nm³] | 62 | 102 |
| SBT [°C] ²) | 360 | 368 |
| HST [°C] | 474 | 462 |
| PSA-Reinausbeute [Gew.-%] | 98 | 99 |

## Patentansprüche

1. Trägerkatalysator für Gasphasenreaktionen mit einem inerten Trägerkörper und einer Oberflächenbeschichtung enthaltend
a) mindestens 5 Gew.% Siliciumcarbid,
b) 5 bis 90 Gew.%, gerechnet als Oxid, einer oder mehrerer Titandioxid- oder Zirkonoxid-Komponenten oder deren Gemische,
c) 1 bis 50 Gew.%, gerechnet als V₂O₅, einer oder mehrerer Vanadiumkomponenten,
d) 0 bis 10 Gew.%, gerechnet als Oxid, einer oder mehrerer Verbindungen von Elementen der 1. und 5. Hauptgruppe des Periodensystems.

2. Trägerkatalysator nach Anspruch 1, dadurch gekennzeichnet, daß 10 bis 75 Gew.%, bezogen auf das Gesamtgewicht der Aktivkomponenten, SiC mit einer Korngröße bis 100 µm enthalten sind.

3. Trägerkatalysator nach Anspruch 1 oder 2, dadurch gekennzeichnet, daß 10 bis 75 Gew.%, bezogen auf das Gesamtgewicht der Aktivkomponenten, TiO₂ enthalten sind.

4. Trägerkatalysator nach Anspruch 1 bis 3, dadurch gekennzeichnet, daß 5 bis 30 Gew.%, bezogen auf das Gesamtgewicht der Aktivkomponenten, V₂O₅ enthalten sind.

5. Trägerkatalysator nach Anspruch 1 bis 4, dadurch gekennzeichnet, daß 0,01 bis 1.0 Gew.%, bezogen auf das Gesamtgewicht der Aktivkomponenten, einer oder mehrerer Verbindungen aus der Gruppe K₂O, Cs₂O, Cs₂CO₃, P₂O₅, (NH₄)₂HPO₄, Sb₂O₃ enthalten sind.

6. Trägerkatalysator nach Anspruch 1 bis 5, dadurch gekennzeichnet, daß 1 bis 30 Gew.%, bezogen auf das Gesamtgewicht des Trägerkatalysators, aktive Masse mit den Aktivkomponenten a) bis d) enthalten sind.

7. Verfahren zur Herstellung von Trägerkatalysatoren nach Anspruch 1 bis 6, dadurch gekennzeichnet, daß die Trägerkörper mit einer wäßrigen Aufschlämmung einer Mischung der Aktivkomponenten oder mit wäßrigen Aufschlämmungen der einzelnen Aktivkomponenten beschichtet und getrocknet werden.

8. Verfahren nach Anspruch 7, dadurch gekennzeichnet, daß eine wäßrige Aufschlämmung, welche die Aktivkomponenten und einen organischen Binder enthält, gleichmäßig auf die Trägerkörper aufgebracht wird.

9. Verwendung des Trägerkatalysators nach Anspruch 1 bis 6 zur Oxidation von Aromaten oder Alkylaromaten sowie deren Gemische zu den entsprechenden Säureanhydriden.

10. Verwendung nach Anspruch 9, dadurch gekennzeichnet, daß die Trägerkatalysatoren bei der Herstellung von Phthalsäureanhydrid durch katalytische Gasphasenoxidation von o-Xylol, Naphthalin oder deren Gemische oder bei der Herstellung von Pyromellithsäuredianhydrid durch katalytische Gasphasenoxidation von 1,2,4,5-tetraalkylierte Benzole verwendet werden.

## Claims

1. Supported catalyst for gas-phase reactions having an inert support body and a surface coating comprising
a) at least 5 % by weight of silicon carbide,
b) from 5 to 90 % by weight, calculated as oxide, of one or more titanium dioxide or zirconium oxide components or mixtures thereof,
c) from 1 to 50 % by weight, calculated as V₂O₅, of one or more vanadium components,
d) from 0 to 10 % by weight, calculated as oxide, of one or more compounds of elements of the 1st and 5th main groups of the Periodic Table.

2. Supported catalyst according to Claim 1, characterized in that from 10 to 75 % by weight, based on the total weight of the active components, of SiC having a particle size of up to 100 µm are present.

3. Supported catalyst according to Claim 1 or 2, characterized in that from 10 to 75 % by weight, based on the total weight of the active components, of TiO₂ are present.

4. Supported catalyst according to any of Claims 1 to 3, characterized in that from 5 to 30 % by weight, based on the total weight of the active components, of V₂O₅ are present.

5. Supported catalyst according to any of Claims 1 to 4, characterized in that from 0.01 to 1.0 % by weight, based on the total weight of the active components, of one or more compounds from the group K₂O, Cs₂O, Cs₂CO₃, P₂O₅, (NH₄)₂HPO₄, Sb₂O₃ is present.

6. Supported catalyst according to any of Claims 1 to 5, characterized in that from 1 to 30 % by weight, based on the total weight of the supported catalyst, of active composition containing the active components a) to d) are present.

7. Process for preparing supported catalysts according to any of Claims 1 to 6, characterized in that the support bodies are coated with an aqueous slurry of a mixture of the active components or with aqueous slurries of the individual active components and are dried.

8. Process according to Claim 7, characterized in that an aqueous slurry containing the active components and an organic binder is applied uniformly to the support bodies.

9. Use of the supported catalyst according to any of Claims 1 to 6 for the oxidation of aromatics or alkyl-aromatics or mixtures thereof to give the corresponding acid anhydrides.

10. Use according to Claim 9, characterized in that the supported catalysts are used in the preparation of phthalic anhydride by catalytic gas-phase oxidation of o-xylene, naphthalene or mixtures thereof or in the preparation of pyromellitic dianhydride by catalytic gas-phase oxidation of 1,2,4,5-tetraalkylated benzenes.

## Revendications

1. Catalyseur sur support pour réactions en phase gazeuse, comportant un corps de support inerte et un revêtement de surface contenant
a) au moins 5% en poids de carbure de silicium;
b) 5 à 90% en poids, calculés comme oxyde, d'un ou de plusieurs composants de dioxyde de titane ou d'oxyde de zirconium ou de leurs mélanges;
c) 1 à 50% en poids, calculés comme V₂O₅, d'un ou de plusieurs composants de vanadium, et
d) 0 à 10% en poids, calculés comme oxyde, d'un ou de plusieurs composés d'éléments des 1er et 5ème groupes principaux du tableau périodique.

2. Catalyseur sur support suivant la revendication 1, caractérisé en ce que sont contenus 10 à 75% en poids, sur la base du poids total des composants actifs, de SiC ayant une taille particulaire jusqu'à 100 µm.

3. Catalyseur sur support suivant la revendication 1 ou 2, caractérisé en ce que sont contenus 10 à 75% en poids, sur la base du poids total des composants actifs, de TiO₂.

4. Catalyseur sur support suivant les revendications 1 à 3, caractérisé en ce que sont contenus 5 à 30% en poids, sur la base du poids total des composants actifs, de V₂O₅.

5. Catalyseur sur support suivant les revendications 1 à 4, caractérisé en ce que sont contenus 0,01 à 1,0% en poids, sur la base du poids total des composants actifs, d'un ou de plusieurs composés du groupe K₂O, Cs₂O, Cs₂CO₃, P₂O₅, (NH₄)₂HPO₄, Sb₂O₃.

6. Catalyseur sur support suivant les revendications 1 à 5, caractérisé en ce que sont contenus 1 à 30% en poids, sur la base du poids total du catalyseur sur support, de composition active comprenant les composants actifs a) à d).

7. Procédé de préparation de catalyseurs sur support suivant les revendications 1 à 6, caractérisé en ce que les corps de support sont revêtus d'une suspension aqueuse d'un mélange des composants actifs ou de suspensions aqueuses des composants actifs séparés, et séchés.

8. Procédé suivant la revendication 7, caractérisé en ce qu'une suspension aqueuse, qui contient les composants actifs et un liant organique, est uniformément appliquée sur le corps de support.

9. Utilisation du catalyseur sur support suivant les revendications 1 à 6, pour l'oxydation d'aromatiques ou alkylaromatiques, ainsi que leurs mélanges, en les anhydrides d'acide correspondants.

10. Utilisation suivant la revendication 9, caractérisée en ce que les catalyseurs sur support sont utilisés lors de la préparation d'anhydride d'acide phtalique par oxydation catalytique en phase gazeuse de o-xylène, de naphtalène ou de leurs mélanges, ou lors de la préparation de dianhydride d'acide pyromellitique par oxydation catalytique en phase gazeuse de benzènes 1,2,4,5-tétraalkylés.
